(19) Europäisches Patentamt / European Patent Office / Office européen des brevets

(11) **EP 4 144 301 A1**

(12) **EUROPEAN PATENT APPLICATION**

(43) Date of publication:
08.03.2023 Bulletin 2023/10

(21) Application number: 21306211.0

(22) Date of filing: 03.09.2021

(51) International Patent Classification (IPC):
*A61B 8/08* (2006.01)     *A61B 8/00* (2006.01)
*G06T 7/00* (2006.01)

(52) Cooperative Patent Classification (CPC):
A61B 8/08; A61B 8/467

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Diagnoly**
**69008 Lyon (FR)**

(72) Inventors:
• **VOZNYUK, Ivan**
 **69008 Lyon (FR)**
• **QUARELLO, Edwin**
 **13008 Marseille (FR)**

(74) Representative: **Icosa**
 **83 avenue Denfert-Rochereau**
 **75014 Paris (FR)**

(54) **DEVICE AND METHOD FOR GUIDING IN ULTRASOUND ASSESSMENT OF AN ORGAN**

(57) The present inveFintion relates to a device (1) for guiding a user in ultrasound assessment of an organ to perform a diagnostic or screening evaluation of said organ during a medical examination, said ultrasound assessment being based on ultrasound images, said device comprising a processor configured to detect and identify the presence of at least one landmark or the absence of landmarks in a current ultrasound image (13); verify whether the identified landmarks in the current image are present in a landmark database (14); if at least one of the identified landmarks in the current image are not present in the landmark database: triggering storage of the current ultrasound image and of the at least one identified landmark which was not comprised in the landmarks database; verifying that all required landmarks have been stored in the landmark database and if at least one of the required landmarks is missing, triggering reception at least one additional current ultrasound image.

FIG. 1

**Description**

**FIELD OF INVENTION**

The present invention relates to the field of medical imaging. In particular, the invention relates to method and system for guiding a user in ultrasound assessment of an organ so as to perform a diagnostic or screening evaluation of said organ during a medical examination.

**BACKGROUND OF INVENTION**

Ultrasound is a non-invasive diagnostic technique used to image inside the body, using sound waves that have frequencies above the threshold of human hearing (above 20KHz). There are many uses of ultrasound, including imaging the heart, blood vessels, eyes, thyroid, brain, breast, abdominal organs, skin, and muscles. According to the body part or the organ under examination, a certain number of landmarks has to be acquired in order for the physician to have sufficient information to perform a correct diagnostic or screening evaluation of said organ. Medical guidelines have defined lists of views (i.e., a planar image of an organ comprising a predefined group of landmarks) specific to each organ, that has to be ideally acquired during an ultrasound acquisition so as to be able to verify the presence or detect the absence of the required landmarks during the examination.

One of the most common uses of ultrasound is during pregnancy, to monitor the growth and development of the fetus by detecting fetal abnormalities. A fetal abnormality is a defect in the fetus of a genetic and/or structural nature that can cause significant fetal health and developmental complications, maternal complicated delivery and lead to complex postnatal medical interventions and adverse outcomes.

Detection of fetal anomalies allows to anticipate a tailored inborn birth, prompt medical care or surgical treatment in specialized centers at birth or in the neonatal period, as well as to provide an adequate information to the parents. For all of these reasons, the ability to detect fetal complications prenatally is critical.

However, despite the progress in equipment, up to 52.8% of prenatal cardiac pathologies are unfortunately not detected today [Pinto2013]. This high rate is due to the fact that being highly "operator-dependent" as well as a "patient-dependent" examination, obstetric ultrasound remains one of the most complex and time-consuming techniques [Pinto2013, Sklansky2016]. As a result, these factors can generate errors in prenatal diagnosis and an overload of work involving a lack of time for health care personnel.

According to specialists [Bensemlali2016, vanNisselrooij2020], fetal heart disease is not always screened antenatally for 3 main reasons: (1) in 49% of cases, lack of skill in adapting ultrasound images (the ultrasound images obtained by the operator are not sufficient to make the correct diagnosis); (2) in 31% of the cases, lack of experience in diagnostics (the images obtained are good and the prenatal pathology is visible but not recognized by the operator); and finally (3), in 20% of cases, pathologies cannot be detected because they are not visible on the ultrasound images.

Different computer-implemented methods have been proposed to help the users to assess fetal ultrasound images and analyze these images in order to detect a large spectrum of abnormalities in fetal development. Most of these methods are focused on the selection of ultrasound images based on their estimated quality. Quality of ultrasound images is estimated on the base of the number of landmarks that should be visible in the view category which the image belongs to. However, obtaining an ultrasound image that comprises all landmarks required for a view by the guideline may be extremely difficult so that images of a view missing a few landmarks are still considered as high-quality images. As a result, these methods allow to select high-quality images which however may not comprise all views or all necessary landmarks which are necessary for a correct medical examination of the organ.

The problem of ensuring the collection of all necessary views for examination have been addressed by patent application EP 20 305 972 of the Applicant. This patent application discloses a method for guiding a user in ultrasound assessment of a fetal organ assisting the user during image acquisition to ensure that all required views of the fetal organ have been acquired. This method analyses all acquired images in order to associate each image to one of the view categories required for a fetal examination according to medical guidelines and evaluate their "quality" according to the number of landmarks that should be visible in the view category to which has been associated. Only images of a high quality are retained and added to a list of valid images to be used for the examination. The user is informed when all the required views have been added to the list of valid images. However, for the user, obtaining images having all or almost all of the landmarks of a guidelines' view may be tricky, and therefore, time consuming for the user and may depend on the expertise of the user.

Furthermore, in the case of examination of the heart, both for fetus, newborn, child or adult, it is of the great interest the possibility of selecting and storing good quality video capturing the dynamic of the organ, which may then be kept as evidence of a medical examination for a predefined lapse of time. In this context, there is the need of developing a method allowing to provide real time analysis of ultrasound video in order to select for storage only few frames but of high quality.

The present invention provides an improved method for guiding a user in ultrasound assessment of an organ so as to perform a diagnostic or screening evaluation of said organ during a medical examination allowing to overcome the draw-backs of the prior art.

## SUMMARY

The present invention relates to device for guiding a user in ultrasound assessment of an organ to perform a diagnostic or screening evaluation of said organ during a medical examination, said ultrasound assessment being based on ultrasound images, said system comprising:

- at least one input configured to receive:

  • a current ultrasound image resulting from at least a partial reflection of ultrasound by said organ;
  • at least two required landmarks used to perform diagnostic or screening evaluation of said organ;
  • a landmarks database, constructed from ultrasound images previously analyzed;

- at least one processor configured to:

  • detect and identify the presence of at least one landmark or the absence of landmarks in the current ultrasound image;
  • verify whether the identified landmarks in the current image are present in the landmark database;
  • if at least one of the identified landmarks in the current image are not present in the landmark database:

    - triggering storage of the current ultrasound image in an image database;
    - triggering storage in the landmark database of the at least one identified landmark which was not comprised in the landmarks database;
    - verifying that all required landmarks have been stored in the landmark database and if at least one of the required landmarks is missing, triggering reception at least one additional current ultrasound image.

The device of the present Invention advantageously allows to analyze in real time the incoming images obtained by an ultrasound probe or an independent set of ultrasound images in order to ensure that all necessary information (i.e., landmarks) have been acquired for ultrasound assessment of an organ. Indeed, the acquisition of all required landmarks is crucial for the user performing the diagnostic or screening evaluation of said organ during a medical examination.

The device of the present invention is particularly advantageous with respect to the method and devices of the prior art which aims at collecting good quality ultrasound images (i.e., image of a view comprising as much of the related landmarks). Indeed, the users (i.e., doctors) do not pursue as aim in real practice to obtain only ideal views, considering that sometimes it is not even possible, due to the technological limitations, operator experience or patient morphology. Thus, the task of evaluating the quality of an ultrasound images with the aim of obtaining a predefined view is highly unpractical. Indeed, the fact that an image has a suboptimal quality (for example comprises only 60% of the landmarks associated to a predefined view) does not mean that said image has no medical value to add to the ultrasound examination. It is therefore much more important to assess the quality of the ultrasound examination (i.e., collection of an ensemble of images comprising the required landmarks disrespect to their presence in a same image corresponding to a predefined view) and not ultrasound views. In fact, for example, an examination may still be considered of good quality since all required landmarks have been acquired in multiple images even if all five images associated to one predefined view have "poor" quality, (e.g., only 40% of the landmarks associated to the view are visible in the image). Thus, the aim of this invention is to provide to the ultrasound user a practically useful tool assessing the quality of the examination without the priority of obtaining a perfect ultrasound view.

According to one embodiment, the at least one processor is configured, further to the detection and identification, to localize the landmarks in the current ultrasound image. This advantageously provides information on the location of the identified landmarks.

According to one embodiment, the at least one processor is further configured to, before the detection of the landmarks, detect a region of interest (ROI) on the current ultrasound image comprising at least a portion, preferably all, anatomical landmarks present in the current ultrasound image. The detection and localization of said ROI is obtained using a machine learning approach. The selection of the ROI comprising all landmarks present in the current image allows to reduce the dimension of the image to analyze and therefore reduce the computational burden and increase the detection precision of the landmarks, especially small ones. This embodiment advantageous facilitates the implantation in real time of the operation of the at least one processor.

According to one embodiment, the at least one processor is further configured to localize the landmarks by using a machine learning approach receiving as input the region of interest of the current ultrasound image.

According to one embodiment, the at least one processor is further configured to compare an area of the region of interest to a predefined threshold and proceed to the localization of the landmarks only if the area of the region of interest exceeds the predefined threshold, otherwise triggering reception of at least one additional current ultrasound image. This embodiment advantageously allows to determine whether the ROI is worth further analysis. Indeed, if the ROI is too small also the landmarks will be too small for further visualization by the user.

According to one embodiment, the at least one processor is further configured to select a machine learning model for performing the detection and identification of the presence or the absence of landmarks in the current ultrasound image. Said machine learning model for performing the detection and identification has been previously trained on ultrasound images comprising at least part of the landmarks comprised in the current ultrasound image. This embodiment advantageously allows to improve the efficiency of identification of landmarks since the current image is analyzed using a dedicated algorithm trained on similar images.

According to one embodiment, the at least one processor is further configured to analyze each localized landmark to evaluate the quality of said localized landmark. This embodiment allows to advantageously ensure that the detected landmarks have a visual quality sufficient for the user to assess the organ and evaluate the eventual presence of a pathology. The quality of the localized landmark may be established on the base of multiple parameters such as the landmark relative size and its orientation, continuity and contrast of the landmark, the clearness of the landmark boundaries and the signal-to-noise ratio.

According to one embodiment, the at least one processor is further configured to trigger storage of the localized landmark in the landmarks database only if the localized landmark has at least a minimal predefined quality. This embodiment allows to advantageously ensure that the stored landmarks have a visual quality sufficient for the user to assess the organ and evaluate the eventual presence of a pathology.

According to one embodiment, the at least one processor is further configured to evaluate a global quality score of the current image when no new landmark is detected in the current image (i.e., all landmarks detected and identified in the current image are already comprised in the landmark database). In this embodiment, the calculated global quality score is compared with the global quality score obtained for the at least one image stored in the image database comprising the same landmarks as the current image. The processor is then configured to trigger the storage of this current image in the image database if its global quality score is higher of the global quality score of the stored image. Notably, the stored image having a lower score may be replaced by the current image (i.e., the stored image is deleted from the image database). This embodiment advantageously allows to ensure that the image having the best global quality for the same group of landmarks is stored in the image database.

According to one embodiment, said global quality score is calculated as a weighted linear combination of the quality parameters calculated for each landmark detected (identified and localized) in the current image.

According to one embodiment, when all required landmarks have been acquired, the at least one processor is further configured to notify the user when all required landmarks have been acquired and stop triggering the reception of additional current ultrasound images. This embodiment allows to advantageously save time for the user by preparing a summary of obtained images and notifying the user that one can stop assessing said organ.

According to one embodiment, the detection and identification of the landmarks is performed by the at least one processor using a machine learning approach comprising at least one Convolutional Neural Network.

According to one embodiment, the at least one processor is configured to iteratively perform the operations to do on a time sequence of ultrasound images.

According to one embodiment, the at least one processor is configured to select a portion of the time sequence of ultrasound images comprising the highest number of localized landmarks having at least a minimal predefined quality. Advantageously, this embodiment allows to select and, therefore store, a good quality video capturing the dynamic of the organ, which may then be keep as evidence of a medical examination for a predefined lapse of time.

All the above described embodiments, may be implemented separately or in combination with one or more other embodiments herein disclosed.

The present invention also relates to method for guiding a user in ultrasound assessment of an organ to perform a diagnostic or screening evaluation of said organ during a medical examination, said ultrasound assessment being based on ultrasound images, said method comprising:

- receiving:

  • a current ultrasound image resulting from at least a partial reflection of ultrasound by said organ;
  • at least two required landmarks used to perform diagnostic or screening evaluation of said organ;
  • a landmarks database, constructed from the ultrasound images previously analyzed;

- detecting and identifying the presence of at least one landmark in the current ultrasound image;
- verifying whether the identified landmarks in the current image are present in the landmark database;
- if at least one of the identified landmarks in the current image are not present in the landmark database:

  • triggering storage of the current ultrasound image in an image database;
  • triggering storage in the landmark database of the at least one identified landmark which was not comprised in the landmarks database;
  • verifying that all required landmarks have been stored in the landmark database and if at least one of the required landmarks is missing, triggering reception at least one additional current ultrasound image.

According to one embodiment, the detection and identification, the at least one processor is further configured to localize the landmarks in the current ultrasound image.

The device for guiding and/or the device for training may be implemented in various ways with proper associated user interface, whether together or separately, notably as a standalone software installed in a computer, a cloud-based service or an Application Programming Interface (API).

In addition, the disclosure relates to a computer program comprising software code adapted to perform a method for guiding or a method for training compliant with any of the above execution modes when the program is executed by a processor.

The present disclosure further pertains to a non-transitory program storage device, readable by a computer, tangibly embodying a program of instructions executable by the computer to perform a method for guiding or a method for training, compliant with the present disclosure.

Such a non-transitory program storage device can be, without limitation, an electronic, magnetic, optical, electromagnetic, infrared, or semiconductor device, or any suitable combination of the foregoing. It is to be appreciated that the following, while providing more specific examples, is merely an illustrative and not exhaustive listing as readily appreciated by one of ordinary skill in the art: a portable computer diskette, a hard disk, a ROM, an EPROM (Erasable Programmable ROM) or a Flash memory, a portable CD-ROM (Compact-Disc ROM).

**DEFINITIONS**

In the present invention, the following terms have the following meanings:

In the present invention, the following terms have the following meanings:

The terms "adapted" and "configured" are used in the present disclosure as broadly encompassing initial configuration, later adaptation or complementation of the present device, or any combination thereof alike, whether effected through material or software means (including firmware).

The term "processor" should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

"Fetus" and "fetal" refers to the unborn offspring of a mammal, preferably a human that develops from an embryo.

"Machine **learning** (ML)" designates in a traditional way computer algorithms improving automatically through experience, on the ground of training data enabling to adjust parameters of computer models through gap reductions between expected outputs extracted from the training data and evaluated outputs computed by the computer models.

"Datasets" are collections of data used to build an ML mathematical model, so as to make data-driven predictions or decisions. In "supervised learning" (i.e. inferring functions from known input-output examples in the form of labelled training data), three types of ML datasets (also designated as ML sets) are typically dedicated to three respective kinds of tasks: "training", i.e. fitting the parameters, "validation", i.e. tuning ML hyperparameters (which are parameters used to control the learning process), and "testing", i.e. checking independently of a training dataset exploited for building a mathematical model that the latter model provides satisfying results.

"Neural network" or "artificial **neural network** (ANN)" designates a category of ML comprising nodes (called neurons), and connections between neurons modeled by "weights". For each neuron, an output is given in function of an input or a set of inputs by an "activation function". Neurons are generally organized into multiple "layers", so that neurons of one layer connect only to neurons of the immediately preceding and immediately following layers.

**"Convolutional neural network"** refers to a neural network which is partly composed of convolutional layers, i.e. layers which apply a convolution on their input. Said series of convolutional layers comprise filters (Kernal) applied to the input data, such as images, in order to automatically extract from them convoluted feature maps. Convolutional neural network may comprise as well different pooling layers, linear and non-linear activation functions and numerical techniques (i.e. batch normalization, dropout, etc) for learning acceleration and stabilization. Very often, convolutional neural networks are connected with fully connected "dense" layers.

**"Fully convolutional neural network"** refers to a convolutional neural network without any fully connected "dense" layers used in the architecture.

**"Landmarks"** refers to any feature consistently present in an organism and possible to be visualized on a medical image. Medical landmarks can be of anatomic or qualitative nature. Independently from physiological or pathological development (i.e., presence of all landmarks is not equal to a physiological development, as well as a lack of some landmarks does not correspond to a pathology), landmarks serve as the principal medical criteria allowing to evaluate given ultrasound image and, especially, given ultrasound examination, from a qualitative viewpoint.

## BRIEF DESCRIPTION OF THE DRAWINGS

Figure 1 is a block diagram representing schematically a particular mode of a device for guiding a user in ultrasound assessment of an organ, compliant with the present disclosure;

Figure 2 schematic representation of a list of some of the ultrasound view categories required by the ISUOG OB/GYN guidelines for the fetal heart examination of the 2nd and 3rd trimesters of pregnancy;

Figure 3 is a flow chart showing successive steps executed with the device for guiding of figure 1;

Figure 4 illustrates a particular implementation of the device for guiding of figure 1, using a combined CNN and FCNN processing for landmarks detection and identification;

Figure 5 is a flow chart showing successive steps executed with the device for guiding of figure 1, according to a specific embodiment wherein the quality of landmarks is evaluated.

Figure 6 illustrates a particular implementation of the device for guiding of figure 1, using a hybrid CNN consisting of 3 connected parts processing for landmarks identification and localization;

Figure 7 illustrates two examples of images of a view C of a fetal heart (i.e., a left ventricular outflow tract), comprising a landmark 3 of the view C;

Figure 8 illustrates two examples of images of a view B of a fetal heart (i.e., four chamber view), comprising a landmark 10 of the view B;

Figure 9 illustrates a detail of exemplary view C of a fetal heart comprising a landmark 5 of the view C;

Figure 10 illustrates two examples of images of a view B of a fetal heart having different visual quality.

Figure 11 is a flow chart showing successive steps executed with the device for guiding of figure 1, according to a specific embodiment wherein the quality of each landmark and the global quality of the current image are evaluated.

Figure 12 is a flow chart successive steps executed with the device for guiding of figure 1, according to a specific embodiment wherein ROI comprising the landmarks is selected for further processing;

Figure 13 illustrates a particular implementation of the device for guiding of figure 1, for identification of a ROI comprising the landmarks is selected for further processing;

Figure 14 is a flow chart successive steps executed with the device for guiding of figure 1, according to a specific embodiment wherein the frame is processed according to the resemblance of the frame to one type of view (i.e., dominant view);

Figure 15 illustrates a particular implementation of the device for guiding of figure 1, for the identification of presence/absence of a dominant view in the current frame;

Figure 16 is a flow chart successive steps executed with the device for guiding of figure 1, according to a specific embodiment wherein a ROI is defined for further processing and the quality of the landmarks, localized in the ROI, is evaluated;

Figure 17 diagrammatically shows an apparatus, comprising at least one graphic processor, integrating the functions of the device for guiding of figure 1 and of the device for training;

Figure 18 diagrammatically shows an apparatus, comprising only CPU, integrating the functions of the device for guiding of figure 1 and of the device for training, according to a specific embodiment.

## ILLUSTRATIVE EMBODIMENTS

The present description illustrates the principles of the present disclosure. It will thus be appreciated that those skilled in the art will be able to devise various arrangements that, although not explicitly described or shown herein, embody the principles of the disclosure and are included within its scope.

All examples and conditional language recited herein are intended for educational purposes to aid the reader in understanding the principles of the disclosure and the concepts contributed by the inventor to furthering the art, and are to be construed as being without limitation to such specifically recited examples and conditions.

Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

Thus, for example, it will be appreciated by those skilled in the art that the block diagrams presented herein may represent conceptual views of illustrative circuitry embodying the principles of the disclosure. Similarly, it will be appreciated that any flow charts, flow diagrams, and the like represent various processes which may be substantially represented in computer readable media and so executed by a computer or processor, whether or not such computer or processor is explicitly shown.

The functions of the various elements shown in the figures may be provided through the use of dedicated hardware as well as hardware capable of executing software in association with appropriate software. When provided by a processor, the functions may be provided by a single dedicated processor, a single shared processor, or a plurality of individual processors, some of which may be shared.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces.

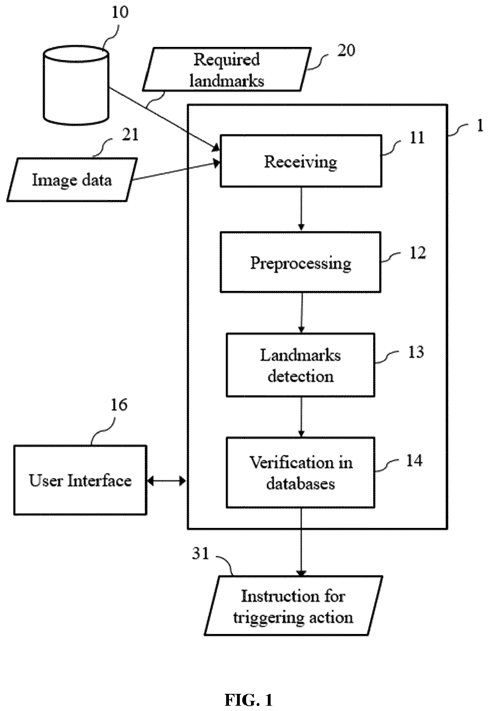

The present disclosure will be described in reference to a particular functional embodiment of a device 1 for guiding a user in ultrasound assessment of an organ, as illustrated on **Figure 1.**

The device 1 is adapted to produce a collection of ultrasound images concerning the organ which together comprise all required landmarks necessary to perform diagnostic or screening evaluation of said organ. In the present disclosure, diagnostic refers to the identification of a pathology while screening refers to a constatation of presence or absence of visually detectable anomalies associated to a pathology. Said collection of ultrasound images is produced from data 20 representative of a time sequence of ultrasound images of one or more organs during an ultrasound assessment (hereinafter "image data").

In advantageous embodiments, the device 1 is further adapted to select a portion of a time sequence of ultrasound images so as to provide a video wherein the images comprise the highest variety of landmarks trying to cover all the requested ones. This gives a highly important medical information mandatory in the assessment of human dynamic organs, such as human heart comprising fetal cardiac examination. Indeed, many pathologies or medical conditions are detectable exclusively in dynamic assessment.

The image data 21 may be derived from a video of an organ, obtained by ultrasound imaging techniques. The device 1 may receive either an image from traditional ultrasound technique or Doppler technique. The image data 21 may be derived from pictures or videos obtained with any ultrasound probe such as Voluson Swift / E8 / E10 (General Electric), EPIQ (Philips Medical), Aplio (Canon Medical), Acuson (Siemens healthineers). They may be periodically time-spaced. In alternative embodiments, the time lapses between at least some of consecutive images may be irregular.

The images may be grayscale images. The image data 21 may include numerical data, such as digital data. Those data may include individual image data in a compressed form, as well known to a person skilled in image compression, such as JFIF/JPG/JPEG (Joint Photographic Experts Group), TIFF (Tagged Image File Format), BMP (Windows Bitmap), PNG (Portable Network Graphics); medical imaging formats such as MINC (Medical Imaging NetCDF) or DICOM (Digital Imaging and Communications in Medicine); or individual image data in a non-compressed form (numerical two- or three-dimensional arrays of uint8 data type ranging from 0 to 255). They may instead, or in addition, include video data in a compressed form, such as MPEG, OGG, MKV, AVI, WMV, GIF, MOV. Those video data may be encoded using any professional codecs (H262, H.264, H265, etc).

The image data 21 may for example comprise between 1 and 120,000 frames, captured during few seconds to 40 minutes at 50 fps (i.e., frame per second), typically 20 minutes at 40 fps.

The device 1 is configured as well to receive as input at least two required landmarks used to perform diagnostic or screening evaluation of said organ. As explained in the background of the invention, the ultrasound examination of an organ is based on the acquisition of a predefined ensemble of views which are defined by medical guidelines. The goal of acquiring all the predefined views for an organ is that of being able to observe at least once all the landmarks that are comprised in the predefined views according to the guidelines. In the present invention, the focus is shifted from the acquisition of the predefined views comprising required landmarks to the acquisition of the required landmarks no matter the number of frames that will be necessary to capture all the landmarks which are usually associated to one single predefined view (i.e., one frame could comprise all required landmarks of a predefined view). Therefore, the list of required landmarks received as input from the device 1 comprises at least a portion, preferably the totality, of the landmarks comprised in the predefined views defined for an organ according to at least one medical guideline.

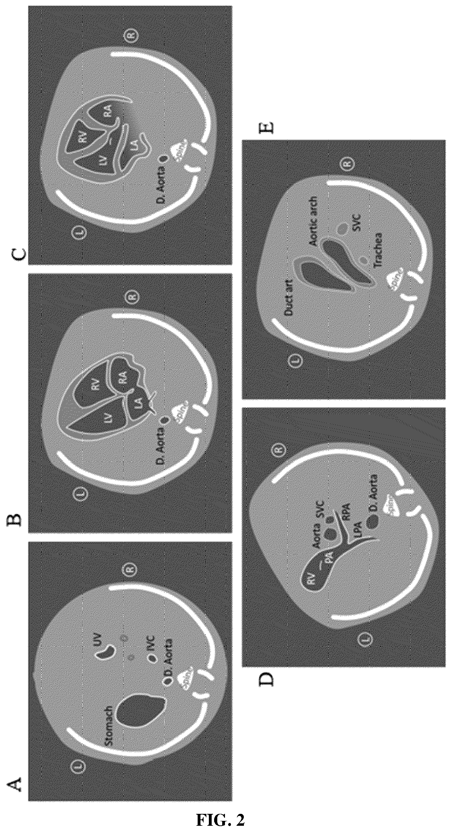

In the case of the organ being a foetus heart, according to the medical guidelines, a four-chamber view comprises the whole heart inside the chest, the size of the picture should take at least half of the screen, and the proportion of the heart, in normal condition, compared to the chest is usually 1/3. Furthermore, in a four-chamber view the following fetal anatomical landmarks should be visible: the apex of the heart, both ventricles if present, the atrio-ventricular valves and their offset, both atria, at least one pulmonary vein, the foramen ovale, and the vieussens valve. An example of a such predefined four-chamber view, corresponding to OB/GYN guidelines, is shown in **Figure 2.**

In one embodiment, the device 1 is configured to receive a list comprising at least two required landmarks associated to the ultrasound views types required, for example by the ISUOG OB/GYN guidelines, for the fetal heart examination of the 2$^{nd}$ and 3$^{rd}$ trimesters of pregnancy. Some of these required ultrasound views types are represented in **Figure** 2. In a preferred embodiment, when the organ under investigation is a foetal heart, the number of required landmarks is comprised between 1 and 57 (view A - 7, view B - 17, view C - 11, view D - 13, view E - 9), preferably 57 (all landmarks required).

The device 1 may be as well configured to receive list of landmarks necessary for the examination of a fetus which are associated also to the brain and central neural system, the skeleton, the face, the urinary apparatus, the digestive apparatus, the pulmonary apparatus and optionally as well the placenta, the umbilical cord and/or the amniotic medium.

The device 1 may as well be configured to assist the user in the assessment of an organ of a newborn, a child or an adult in order to evaluate pain, swelling, infection or a pathology. In this case, the list of landmarks that is received may comprise landmarks for heart and blood vessels, liver, kidneys, uterus and ovaries, eyes, thyroid and parathyroid glands, and scrotum.

Furthermore, the device 1 is configured to receive as input a landmarks database, constructed from ultrasound images previously analyzed. The landmarks database may be as well a list of landmarks constructed from ultrasound images previously analyzed, stored in a database 10, described below. Said landmarks database comprises the required landmarks that have been detected and identified in a previous iteration of the processor. When the ultrasound assessment starts (i.e., acquisition of the first ultrasound frame and reception of the first current image of the assessment by the device, in case of real time, or reception of the first current image of the assessment by the device, in case of off-line analysis), the landmarks database is empty. The content of the landmarks database is increased as new current images are analyzed by the processor as described in further details below.

The device 1 for predicting is associated with a training device for training suited to setting model parameters of the device 1 in a proper way.

Though the presently described devices 1 and the training device are versatile and provided with several functions that can be carried out alternatively or in any cumulative way, other implementations within the scope of the present disclosure include devices having only parts of the present functionalities.

Each of the devices 1 and the training device is advantageously an apparatus, or a physical part of an apparatus, designed, configured and/or adapted for performing the mentioned functions and producing the mentioned effects or results. In alternative implementations, any of the device 1 and the training device is embodied as a set of apparatus or physical parts of apparatus, whether grouped in a same machine or in different, possibly remote, machines. The device 1 and/or the training device may e.g. have functions distributed over a cloud infrastructure and be available to users as a cloud-based service, or have remote functions accessible through an API.

The device 1 for guiding and the device for training may be integrated in a same apparatus or set of apparatus, and intended to same users. In other implementations, the structure of device for training may be completely independent of the structure of device 1, and may be provided for other users. For example, the device 1 may have a parameterized model available to operators for guiding a user in ultrasound assessment of an organ, wholly set from previous training effected upstream by other users with the device for training.

In what follows, the modules are to be understood as functional entities rather than material, physically distinct, components. They can consequently be embodied either as grouped together in the same tangible and concrete component, or distributed into several such components. Also, each of those modules is possibly itself shared between at least two physical components. In addition, the modules are implemented in hardware, software, firmware, or any mixed form thereof as well. They are preferably embodied within at least one processor of the device 1 or of the device for training.

The device 1 comprises a module 11 for receiving the required landmarks 20, the image 21 and the landmark database 22 (i.e., list of already detected landmarks) stored in one or more local or remote database(s) 10. The receiving module 11 may be as well configured to receive ML (machine learning) parameters. The latter can take the form of storage resources available from any kind of appropriate storage means, which can be notably a RAM or an EEPROM (Electrically-Erasable Programmable Read-Only Memory) such as a Flash memory, possibly within an SSD (Solid-State Disk). In advantageous embodiments, the ML parameters 20 have been previously generated by a system including the device for training. Alternatively, the ML parameters 20 are received from a communication network.

The device 1 further comprises optionally a module 12 for preprocessing the received image data 21. The module 12 may notably be adapted to standardize the received image data 21 for sake of efficient and reliable processing. It may transform the image data 21, e.g. by image or video decompression. It may extract features from the image data 21. According to various configurations, the module 12 is adapted to execute only part or all of the above functions, in any possible combination, in any manner suited to the following processing stage.

In advantageous modes, the module 12 is configured for preprocessing the image data 21 so as to have the images standardized. This may enhance the efficiency of the downstream processing by the device 1. Such a standardization may be particularly useful when exploited images originate from different sources, including possibly different imaging systems.

The standardization is advantageously applied to the image data 21 and to image sequences of training datasets (e.g. by the device 6) in a similar way. Notably, the device 1 may deal with image data coming from a given sort of source, while its parameters result from a training based on image data obtained with different types of sources. Thanks to standardization, differences between sources may then be neutralized or minimized. This may make the device 1 more efficient and reliable.

In one example, the module 12 may be configured to decompress frame to a 3d uint8 array of size HxWx3, where H is image height, W is image width and 3 corresponds to RGB channels. Once decompressed, the frame is cropped in order to remove the patient information bars (depending on the ultrasound system type). Module 12 may as well detect if the image is simple (contains a single view plane), doubled (in Doppler technique) or doubled (contains two different independent views), which provides an information of interest for the post processing of the frame. The frame is as well processed to improve image qualitatively (i.e., by noise reduction) and quantitatively (i.e., by ultrasound artefacts reduction, such as ultrasound shadows, acoustic destructive interferences). Finally, the frame may be scaled to size 480x640 or 640x640 and the type of the image may be changed to a float16 (half float precision) and normalize into the range 0 to 1, or -1 to 1.

The device 1 comprises a module 13 configured for detecting and identifying the presence of at least one landmark or the absence of landmarks in the current ultrasound image (i.e., image data 21).

It may be observed that the operations by the modules 11, 12 and 13 are not necessarily successive in time, and may overlap, proceed in parallel or alternate, in any appropriate manner, notably when image data are analyzed off-line. For example, new image data may be progressively received over time and preprocessed, while the module 13 is dealing with the previously obtained image data. In alternative examples, a batch of image data 21 corresponding to a complete ultrasound sequence comprising multiple frames may be fully received and preprocessed before it is submitted to the module 13.

The device 1 further comprises a module 14 for extracting the instructions for triggering an action 31 from the step outputs executed by the module 13 and for outputting those the instructions for triggering an action 31. The actions that may be triggered by module 14 depends on the state of the landmarks database and whether or not all required landmarks have been stored in it.

The device 1 is interacting with a user interface 16, via which information can be entered and retrieved by a user. The user interface 16 includes any means appropriate for entering or retrieving data, information or instructions, notably visual, tactile and/or audio capacities that can encompass any or several of the following means as well known by a person skilled in the art: a screen, a keyboard, a trackball, a touchpad, a touchscreen, a loudspeaker, a voice recognition system.

In its automatic actions, the device 1 may for example execute the following process:

- receiving:

    - a current ultrasound image resulting from at least a partial reflection of ultrasound by said organ;
    - at least two required landmarks used to perform diagnostic or screening evaluation of said organ;
    - a landmarks database, constructed from the ultrasound images previously analyzed;

- detecting and identifying the present of at least one landmark in the current ultrasound image;
- verifying whether the identified landmarks in the current image are present in the landmark database;
- if at least one of the identified landmarks in the current image are not present in the landmark database:

    - triggering storage of the current ultrasound image in an image database;
    - triggering storage in the landmark database of the at least one identified landmark which was not comprised in the landmarks database;
    - verifying that all required landmarks have been stored in the landmark database and if at least one of the required landmarks is missing, triggering reception at least one additional current ultrasound image.

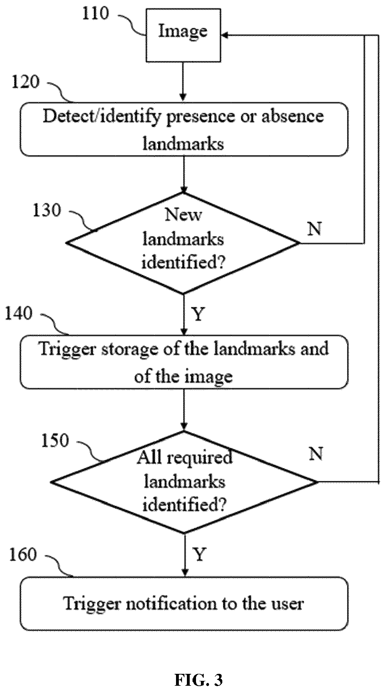

In particular, the device 1 may for example execute the processes illustrated in **Figures 3, 5, 6, 7, 12, 14** and **16,** which will be discussed in detail here below.

A particularly efficient implementation of the device 1 for predicting will be developed in relation with **Figure 3**, showing an unfolded representation of the steps performed by the different modules. In this first embodiment, images 110 are processed in a chronological order according to their acquisition and module 13 is configured to detect and identify the presence of at least one landmark or the absence of landmarks in the current ultrasound image. Module 14 is then configured to verify whether the at least one identified landmark in the current image are present in the landmark database 130.

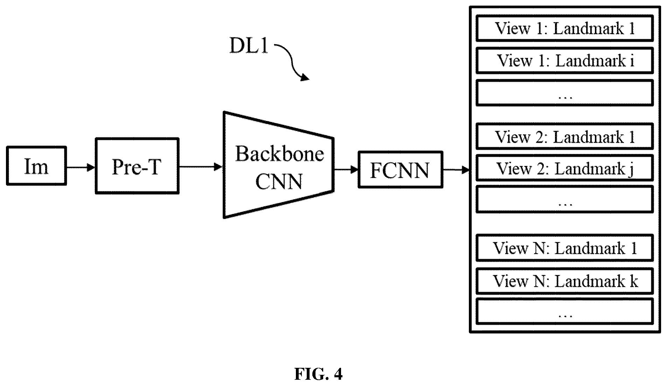

Module 13 may use a machine learning approach for the detection and identification of the landmarks. With reference to **Figure 4,** said machine learning approach has a structure DL1 comprising a first stage employing a convolutional neural network (CNN).

Advantageously, CNN architecture showed its efficiency for the tasks related to computer vision and image treatment as a very powerful and efficient model performing automatic feature extraction to achieve superhuman accuracy. More in details, CNN architecture presents many advantages compared with its predecessors. The filters of CNNs are designed to automatically extract and learn important features from the images. CNN architectures treat directly 2D and 3D data which could be gray-scale and color images and are much more computationally efficient (in terms of number of trainable parameters, weights, memory etc.). As a result, the CNN architectures provide higher prediction accuracy on tasks related to computer vision, image/videos treatments, etc.

The image analysis structure DL1 may further comprise at said first stage bias layer, max pooling layers, batch normalization and/or activation layers. The first stage of DL1 may comprise, before the convolutional neural network, a pre-processing the image, for example for denoising the image.

The machine learning approach, further implies according to this example, a second stage employing a fully connected neural network (FCNN) receiving as input at least a portion of the output of the first stage. In one example, for each provided image the classifier DL1 provides as output a vector of size M, where M corresponds to the total number of anatomical/qualitative landmarks associated to the organ. The landmarks present on the provided image are defined using the following mathematical rule: *where(vector>0)*.

For the training of the classifier DL1 is performed using an activation function merged with a scaling function from 0 to 1, or a simple tanh or sigmoid activation. For these two cases, a cross entropy loss function is used.

If at least one new landmark has been detected and identified, module 14 outputs an instruction for triggering storage of the image 110 in an image database and triggering storage in the landmark database of the at least one identified landmark which was not comprised in the landmarks database.

Otherwise, module 14 outputs an instruction configured to trigger the reception of a new image 110. In the case of a real time ultrasound assessment, the device 1 may for example visualize, via the interface 16, a red spot indicating to the user that the assessment should continue since at least one new image is required. Alternatively, in the case of use of the device 1 for an off-line mode, the instruction will result in triggering the reception of a new image 110 which follows chronologically the image previously analyzed. The images may be retrieved from a sequence of ultrasound image frames chronologically ordered previously stored in the database 10.

Finally, in this example, each time that at least one new identified landmark is added to the landmarks database, the module 14 is configured to verify if all the required landmarks 20 are currently comprised in the database 150. If the landmark database comprises all the required landmarks 21, module 14 triggers notifying the used that all required landmarks have been acquired and may stop triggering the reception of additional images 110. In a real-time assessment, the notification may be provided to the user as a written message indicating that the assessment may be stopped as all the required landmarks have been finally acquired. During an off-line analysis, the device 1 will stop receiving new images 110 from the database 10.

Inversely, if one or more required landmarks are still missing from the landmark database, the module 14 triggers the reception of a new image 110.

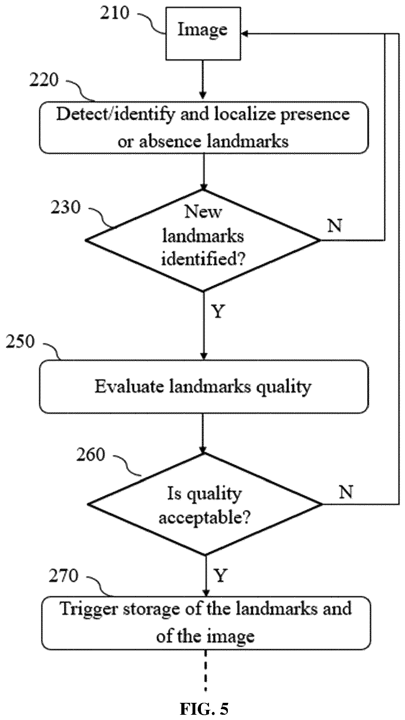

Another embodiment of the invention is present in **Figure** 5 wherein module 13 is further configured to localize the landmarks in the current ultrasound image conjointly or subsequently to their detection and identification. Module 13 may use a machine learning approach configured to receive as input the image 210 and provide an output concerning the detection/identification (i.e., information associated to which landmark of a predefined view according to the guidelines corresponds to the detected landmark) and localization of the landmark. In one preferred embodiment, the information on the localization comprises coordinates and dimensions of a bounding box comprising the landmark. The structure DL1 described before may be further configured to provide as output the localization of the landmarks. In one embodiment, a structure DL2 configured to provide as output the localization of the landmarks is an object detection algorithm, such as Fast R-FCN architecture which uses a region of interest for the convolution.

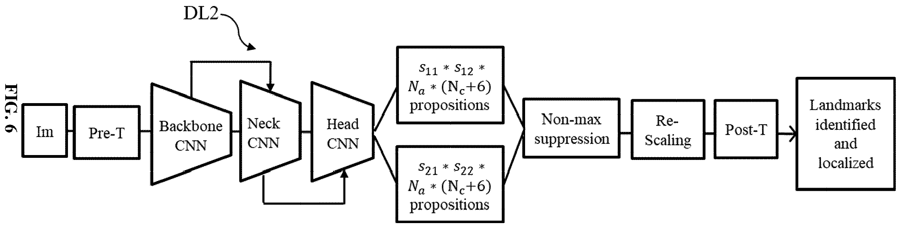

One example of structure that may be used for detection, identification and localization of landmarks is illustrated in **Figure** 6. In this example, the DL2 structure is a YOLO ("You only look once") architecture which uses a region of interest for the convolution. This object detection structure has a single-stage end-to-end trained architecture consisting of three deep learning parts: model backbone, model neck, and model head.

The backbone model is an architecture used to extract the important features from the current ultrasound image 21. This backbone model is based on the Cross Stage Partial Network which is pretrained using transfer learning technique from the dominant view classification task, that will be described below.

The model neck is an architecture used for feature aggregation via the generation of feature pyramids. This part of the architecture makes the algorithm agnostic with respect to the object scaling (i.e., same objects of different size and scale are identified in the same way). The model neck may be chosen among the following algorithms: FPN variations (traditional FPN, NAS-FPN, BiFPN, etc), ASFF, etc. In a preferred embodiment the model neck is based on an Aggregation Network for Instance Segmentation (PANet) network architecture, which was implemented for generating ultrasound feature pyramids.

Model head is a network architecture consuming features from the model neck and performing the final dense predictions: detection and classification of the boxes. The corresponding architecture may be fully-connected neural network, convolutional, or fully-convolutional. The output of the model head depends on a grid applied to the initial current ultrasound image. The initial current ultrasound image is split into two grids of shapes $(s_{11}, s_{12})$ and $(s_{21}, s_{22})$. Each grid cell has $N_a$ anchors allowing $N_a$ bounding boxes. For each of the bounding boxes, the network architecture of the model head outputs a vector of size $(N_c + 6)$ containing $N_c$ class probabilities and 6 main values for the bounding boxes: local center coordinates (x and y), box height and width, rotation angle and confidence score. As a result, the size of the final output layer values is equal to $(s_{11} * s_{12} + s_{21} * s_{22}) * N_a * (N_c + 6)$.

This structure is based on neural network architectures of the type specific for image analysis and object detection.

As for the previous embodiment, the identified landmarks are compared to the landmarks in the landmarks database 230. In this example, whenever a new landmark is detected and identified, its quality is evaluated. In this case the Applicant refers to the visual quality of the landmark in the ultrasound frame, which may be measured by evaluating at least one quality parameter such as the landmark relative size and its orientation, continuity and contrast of the landmark, the clearness of the landmark boundaries. The quality of each landmark may be evaluated by analyzing the portion of ultrasound frame comprised in the bounding box associated to the landmark at step 220.

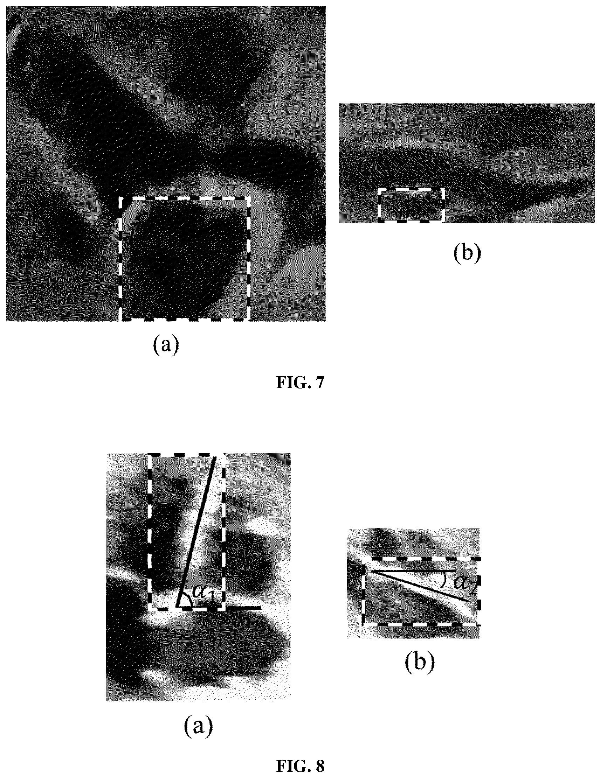

Some examples of quality parameters are visualized in the following **Figures** 7-10. **Figure** 7(a) **and** 7(b), show two images of a view C (i.e., left ventricular outflow tract), comprising a landmark 3. Said landmark 3 corresponds to an anechogenic space located below the semilunar valves, corresponding to the atrium located on the left and behind the left atrioventricular valve. **Figure** 7(a) presents a landmark 3 of larger size while **Figure** 7(b) present a landmark 3 of small size. This example represents an illustration of the relation between the size of the landmarks and their visual quality as it is evident that the landmark of **Figure** 7(b) is therefore associated to a poor visual quality compared to the one of **Figure** 7(a).

**Figure** 8(a) and 8(b) shows two images of view B (i.e., four chamber view) comprising a landmark 10 associated to the connection between the ventricular septum and the crux. The quality of this landmark directly depends on the angle on which this landmark is obtained. In **Figure** 8(a) the angle of this landmark is -80deg, thus this landmark is not of a good quality while in **Figure** 8(b) the angle of this landmark is -350deg, which is associated to a good quality for this landmark.

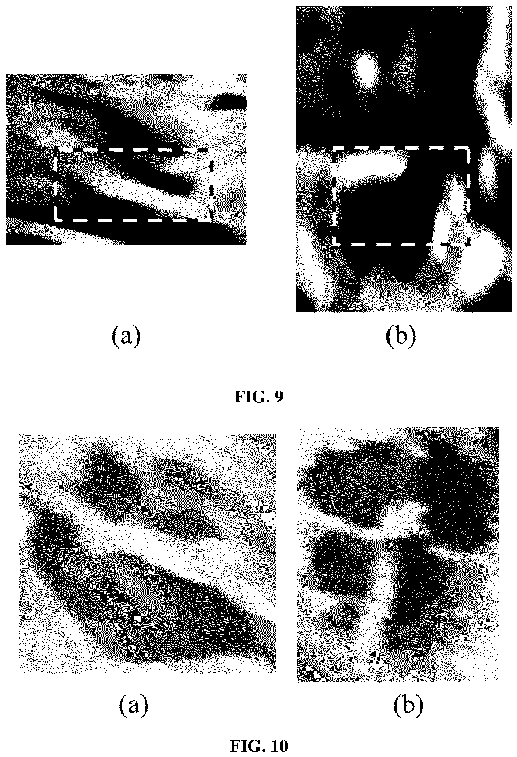

**Figure** 9(a) and 9(b) shows two images of view C comprising a landmark 5 representing the relationship between the interventricular septum and the overlying vessel. In **Figure** 9(a) the landmark 5 is continuous, which is associated to a good visual quality, while in **Figure** 9(b) the landmark 5 is not continuous and therefore it is associated to a poor quality.

Acquisition of a high-quality landmark advantageously allows to reduce significantly the risk of missing a pathology related to the landmark under analysis. As an illustrative example of the importance of the landmark's quality evaluation, we can consider the landmark associated to the interventricular septum, present on the four-chamber view. On **Figure** 8(a), the presence of this landmark is clearly visible, however this landmark is not continuous. Indeed, the hole present in the interventricular septum (near crux) may be of a pathological nature (for example, ventricular septal defect, opening in the wall dividing the left and right ventricles of the heart). In order to validate this landmark, the user should rotate the ultrasound probe perpendicularly to the interventricular septum (this defines the importance of the landmark's orientation). In this case, the detected and localized landmark will be then evaluated for the continuity and clearness. These three quality criteria will define the final quality estimation of the landmark. If the final quality is high (as, for example, on Figure 10) the user reviewing the selected ultrasound images during the examination can ensure a correct evolution of the presence or not of pathologies related to the interventricular septum, such as ventricular septal defect.

Figure 10(a) and 10(b) provides an example of good and bad images dedicated to detect a possible pathology. These images, notably provide a comparison of the image qualities for evaluating the presence or absence of ventricular septal defect. Figure 10(a) is associated to high quality, because the landmark 10 is visible, continuous and under an acceptable angle while Figure 10(b) is associated to low quality, because landmark 10, even if clearly visible, is not continuous and not under an acceptable angle.

The quality parameter(s) evaluated are then used to establish if the visual quality of the landmark is acceptable 260 for providing meaningful information to the user visualizing it during the examination of the organ. Each quality parameter may be compared to a predefined threshold for the quality parameter so that only landmarks having all quality parameters exceeding the predefined threshold are considered to have acceptable quality.

In one embodiment, the quality may be evaluated only for the landmarks of the image 210 which are not already comprised in the landmark database in order to reduce the calculation to perform. Alternatively, the quality may be evaluated for all the landmarks in the image 210, even when only one of the detected landmarks is not included in the landmarks database.

When the quality of the landmark is considered acceptable, the module 15 outputs an instruction configured to trigger the storage of the accepted landmark in the landmark database and the storage of the image in the image database 270. The steps 250 and 260 are performed for all new landmarks detected and identified in the image 210. These actions 250 and 260 may be performed consecutively or in parallel. However, whenever more than one newly identified landmark is acceptable for its quality, the image comprising these newly identified landmarks may be added only once in the image database.

According to one embodiment, the evaluation of the landmark quality (250) is performed before the step 230 of evaluating whether or not a new landmark has been detected.

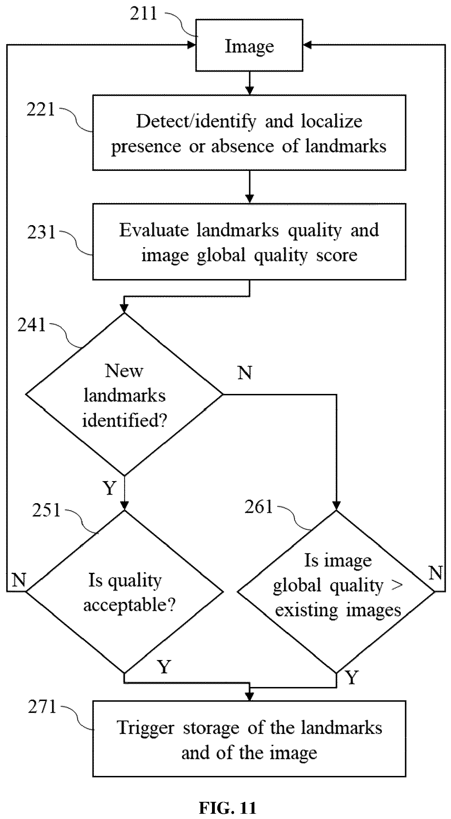

As illustrated in the example of **Figure 11,** the module 15 may be as well configured, in addition to the calculation of landmarks quality, to calculate a global quality score combining the quality parameters obtained for each landmark detected in the image 21 (see step 231). In this example, the step 231 of evaluating the quality of the landmarks and calculating the global quality of the image is performed before the step 241 of comparing the localized landmarks of the current image to the landmarks database for the identification of new landmarks.

Said global quality score may be calculated as a weighted linear combination according to the following formula:

$$\frac{\sum_{l=0}^{N_l} \alpha_l \sum_{\forall i \in m_l} w_i p_i}{N_l N_{m_l}}$$

where $l$ is the index of the landmark, $\alpha_l$ is a binary value configured to be equal to 1 when the landmark is present in the view and to be equal 0 otherwise, i is the index of the quality parameter, $m_l$ is the list of quality parameters applicable to the landmark $l$, $w_i$ is the weight corresponding to the given quality parameter, $p_i$ is the normalized value of the quality parameter, finally, $N_l$ and $N_{m_l}$ are respectively the number of landmarks corresponding to one given ultrasound view and the number of quality parameters applicable to the landmark $l$. Other way of combining the quality parameters in other to obtain an evaluation of the visual global quality, known by the man skilled in the art, may be used.

The resulting value will be stored with the image if new landmarks with acceptable quality have been identified (steps 241,251) and, therefore, the storage of the image has been triggered.

Alternatively, if no new landmark has been identified at step 241, the global quality score may be used at block 261. This step 261 is configured to compare the global quality score of the current image 211 with the global quality score of at least one image already stored in the image database which has the same landmarks of the current image 211. If the current image 211 has a global quality score higher than the global quality score of the image in the image database, then the module 15 is configured to trigger the storage of the current image 211, and optionally to delete the image in the database having a lower global quality score. This advantageously allows to verify if the new image is "better" than the one that is already stored in the memory.

For example, assuming that an image (IM1) with landmarks 1, 2, 3 and 4, of the overall quality score 0.8, have been obtained and stored and at a second iteration of the method a second image (IM2) with landmarks 1,2,4 and 5 is received as input. For this current image the landmarks quality and global quality will be calculated, and if quality of landmark 5 is acceptable the image (IM2) will be stored in the image database and the landmark 5 in the landmarks database. A third image (IM3) with landmarks 1, 2, 3 and 4 of the global quality score 0.85, is received. In this case, no new landmark is detected, but the global quality score of the IM3 is higher than for the IM1 (containing the same landmarks), therefore the third image (IM3) will replace the first image (IM1).

Finally, if we obtain a new image IM4 with landmarks 1,2,3,4 and 5, this image will replace the third image (if score of IM4>IM3) and will replace the second image (if score of IM4>IM2). In our example, if the new image IM4 has a score 0.9, it will replace image IM3 and IM2, because IM4 covers all landmarks of these two images.

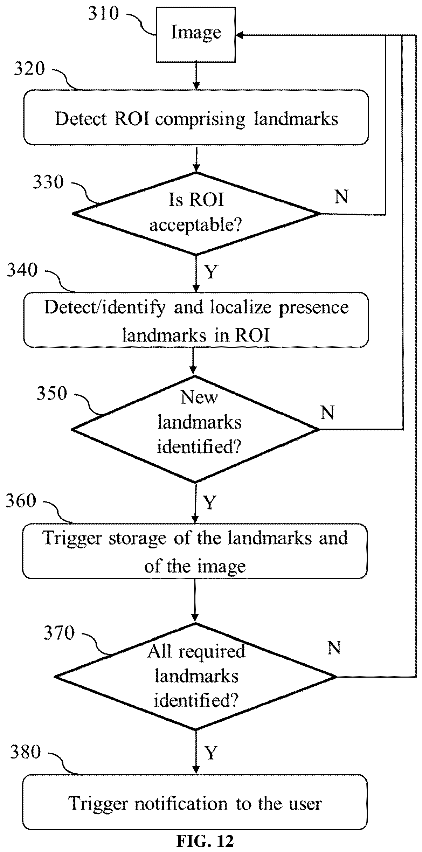

**Figure 12** presents another advantageous embodiment wherein the at least one processor is further configured to detect a region of interest (ROI) on the image 310 comprising all identified landmarks present in the image 320. The advantage of detecting at first this ROI comprising all the meaningful information of the image 310, is that the following steps may be performed by the processor exclusively on this ROI, instead that on the whole image, which reduces the computational time and significantly increases the final precision.

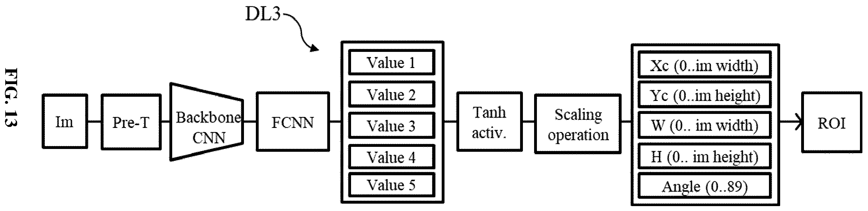

The detection of the ROI may be performed using a machine learning approach. An example of the structure of a machine learning approach is represented in **Figure 13.** In this example the machine learning approach is based on a neural network architecture (DL3). The image 310 is provided as input to a first stage configured to perform a pre-treatment of the image comprising at least one of the following operations of cropping, scaling and padding, detecting the image type (i.e., simple image, doppler image, two different images on the same frame), improving qualitatively and quantitatively the image, and/or normalizing the image. Then the pre-treated image is processed by a convolutional neural network (CNN) followed by a fully connected regression neural network configured to provide as output five continuous values. A tanh activation operation layer (from -1 to 1, or from 0 to 1) and a scaling operator (for values 1 and 3 - from 0 to the image width, for values 2 and 4 - from 0 to the image height, for value 5 - from 0 to 89 degrees) are than configured to use the result of the regression algorithm to provide the coordinates and orientation of the bounding box defining the global ROI comprising all landmarks present in the image 310. Optionally, a confidence score may also be added for this network. For the training of this neural network architecture, it is possible to apply a Huber Loss or a Log-Cosh Loss functions, which advantageously allows to provide more accurate results than Mean Square/Absolute. Optionally, quantile loss functions may also be used for this type of application.

The at least one processor is further configured to evaluate whether the dimension or quality (for example the signal-to-noise ratio) of the defined ROI are acceptable 330 to be able to perform a correct further processing of the image 310. An area of the ROI may be compared to a predefined threshold, and so that the processor proceeds to the localization of the landmarks only if the area of the ROI exceeds the predefined threshold. Otherwise, the processor will trigger the reception of at least one additional image 310. The area of the ROI may be for example evaluated as the number of pixels comprised in the ROI or any other measure known by the person skilled in the art.

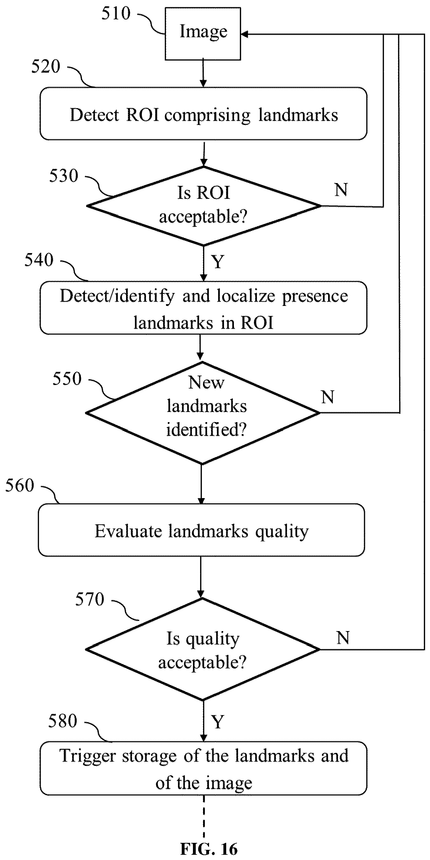

In this example the step of detection and identification of the landmarks 340 is performed only in the ROI. When the step of detection and identification also comprises localization **(Fig. 16),** these operations may all be performed on the ROI. As disclosed before, the detection and identification and localization may be performed by machine learning approach, notably using the example already described.

As for the embodiment presented above, the identified landmarks are compared to the landmarks in the landmarks database 350 which will be stored only if they were not already comprised in the landmarks database 360. As before, satisfying the condition of having a landmark database comprising all required landmarks 370 will trigger the notification informing the user 380. Otherwise, the processor will trigger the reception of a new image 310.

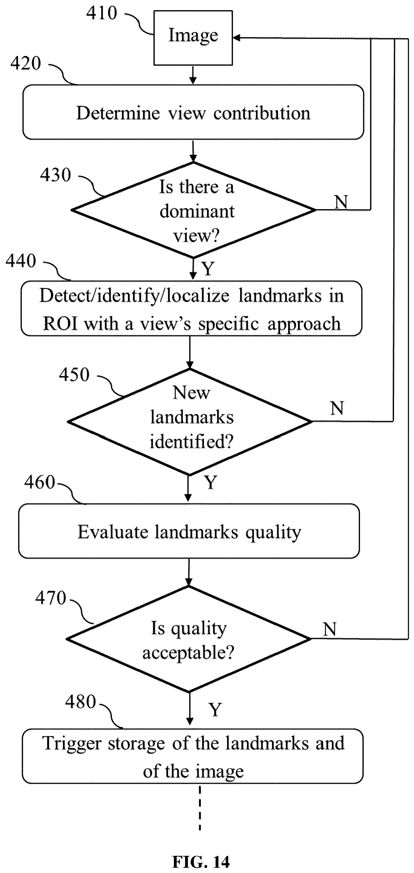

**Figure 14** provides an illustration of an advantageous embodiment wherein the image 410 is at first classified according to its similarity to a predefined set of views 420. The determination of the percentage of similarity between the image 410 and the view of the predefined set of view may be performed by machine learning approach.

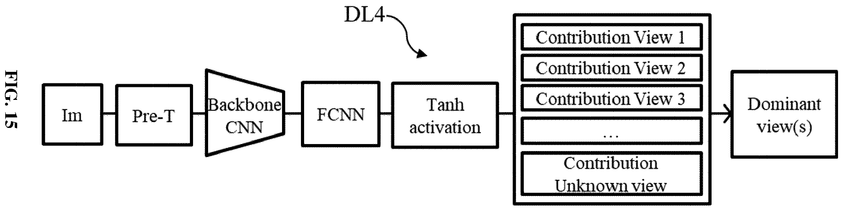

The machine learning approach may rely on a neural network architecture (DL4) represented in **Figure 15**. This architecture comprises a pre-treatment stage configured to apply at least one of the following operations to the image 410: cropping, scaling and padding, detecting the image type (i.e., simple image, doppler image, two different images on the same frame, signal extraction), improving qualitatively and quantitatively the image, and/or normalizing the image. The pre-treated image is then transferred to a backbone (i.e., the feature extracting network) CNN followed by a fully connected neural network. The specificity of this network DL4 is that the result is not a discrete one-hot activation layer. Indeed, the given image may be located between multiple views. Softmax activation function, which is largely used in research cannot thus be used for this specific network and is thus replaced by a hybrid tanh activation function. The output of the tanh activation function provides a percentage representative of the contribution of each view of the image 410. This information is then used to determine a dominant view (i.e., the classifier associated to one view class having a probability superior for example of 50%).

Cross entropy loss function applied to the activation layer may be used for training this structure. The training dataset for this neural network comprises images labelled according to their similarity to one or more views of the predetermined set of view which has been defined according to the clinical guidelines for the specific organ.

If a dominant view has been detected, then this information is used for the following step in order to choose a more suitable machine learning approach that has been specifically trained on images associated to the same view class (i.e., ultrasound images comprising at least part of the landmarks). This approach, thanks to *ad hoc* choice of the used model, provides a higher sensitivity.

In the illustrated example, step 440 of detecting and identifying (or detecting, identifying and localizing) is therefore performed on a machine learning approach specific to the dominant view detected at step 430. This structure may be a neural network architecture of the type described above (DL2) which however, instead of having been trained on all types of images, it has been trained only on images comprising a same or approximately same ensemble of landmarks specific to a predefined view. The steps that follow concerning the check for identification of new landmarks and the quality evaluation of these new landmarks (450, 460 etc.) may be implemented as for the examples described above.

In one embodiment, the machine learning model for detecting, identifying and localizing may be applied on a ROI which comprised the landmarks of the dominant view.

The embodiments of **Figures 12,14,16** may be combined with the embodiments of **Figure 11** concerning the evaluation of the global score of the current image and its comparison with the at least one image of the image database having the same detected landmarks. As explained above, for all the embodiments presented in these figures, the step of evaluating landmarks quality (with the calculation of the global quality score) may be implemented before the verification of the presence of new landmarks in the current image.

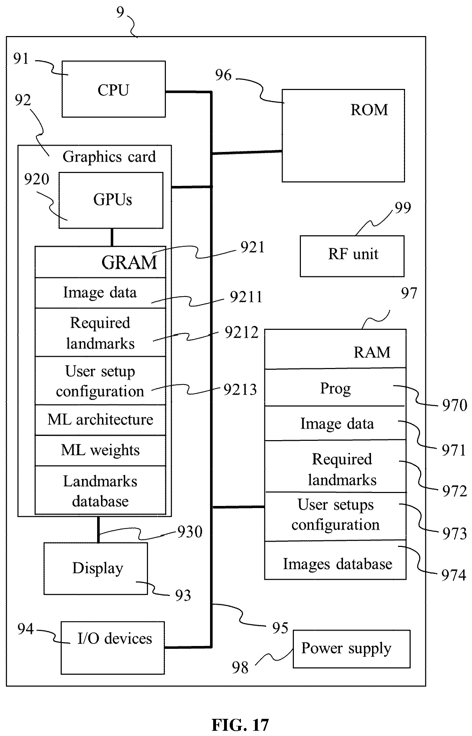

A particular apparatus 9, visible on **Figure 17,** is embodying the device 1 as well as the device for training described above. It corresponds, for example, to a workstation, a laptop, a tablet, a smartphone, or a head-mounted display (HMD).

That apparatus 9 is suited for guiding a user in ultrasound assessment of an organ and to related ML training. It comprises the following elements, connected to each other by a bus 95 of addresses and data that also transports a clock signal:

- a microprocessor 91 (or CPU);
- a graphics card 92 comprising several Graphical Processing Units (or GPUs) 920 and a Graphical Random Access Memory (GRAM) 921; the GPUs are quite suited to image processing by e.g. the CNN block, due to their highly parallel structure;
- a non-volatile memory of ROM type 96;
- a RAM 97;
- one or several I/O (Input/Output) devices 94 such as for example a keyboard, a mouse, a trackball, a webcam; other modes for introduction of commands such as for example vocal recognition are also possible;
- a power source 98; and
- a radiofrequency unit 99.

According to a variant, the power supply 98 is external to the apparatus 9.

The apparatus 9 also comprises a display device 93 of display screen type directly connected to the graphics card 92 to display synthesized images calculated and composed in the graphics card. The use of a dedicated bus to connect the display device 93 to the graphics card 92 offers the advantage of having much greater data transmission bitrates and thus reducing the latency time for the displaying of images composed by the graphics card, for example for identifying and localizing the landmarks. According to a variant, a display device is external to apparatus 9 and is connected thereto by a cable or wirelessly for transmitting the display signals. The apparatus 9, for example through the graphics card 92, comprises an interface for transmission or connection adapted to transmit a display signal to an external display means such as for example an LCD or plasma screen or a video-projector. In this respect, the RF unit 99 can be used for wireless transmissions.

It is noted that the word "register" used hereinafter in the description of memories 97 and 921 can designate in each of the memories mentioned, a memory zone of low capacity (some binary data) as well as a memory zone of large capacity (enabling a whole program to be stored or all or part of the data representative of data calculated or to be displayed). Also, the registers represented for the RAM 97 and the GRAM 921 can be arranged and constituted in any manner, and each of them does not necessarily correspond to adjacent memory locations and can be distributed otherwise (which covers notably the situation in which one register includes several smaller registers).

When switched-on, the microprocessor 91 loads and executes the instructions of the program contained in the RAM 97.

As will be understood by a skilled person, the presence of the graphics card 92 is not mandatory, and can be replaced with entire CPU processing and/or simpler visualization implementations.

In variant modes, the apparatus 9 may include only the functionalities of the device 1, and not the learning capacities of the device for training. In addition, the device 1 and/or the device for training may be implemented differently than a standalone software, and an apparatus or set of apparatus comprising only parts of the apparatus 9 may be exploited through an API call or via a cloud interface.

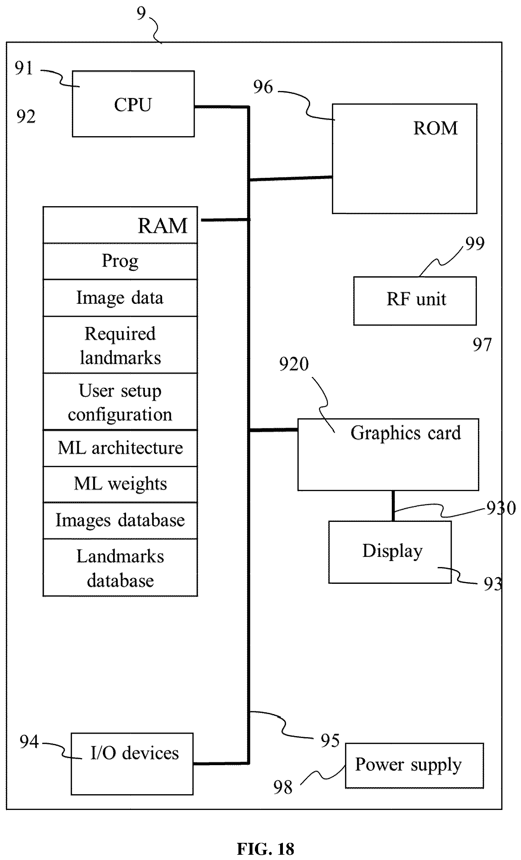

**Figure 18** shows an alternative example for the apparatus 9 comprising an appropriate GPU (one that can be used for ML calculation). For example, a simple video card may be integrated into processor. It has no CUDA technology able to paralyze numerical computations.

**Claims**

1. A device (1) for guiding a user in ultrasound assessment of an organ to perform a diagnostic or screening evaluation of said organ during a medical examination, said ultrasound assessment being based on ultrasound images, said system comprising:

    - at least one input configured to receive (11):

        o a current ultrasound image (20) resulting from at least a partial reflection of ultrasound by said organ;
        o at least two required landmarks (21) used to perform diagnostic or screening evaluation of said organ;
        o a landmarks database, constructed from ultrasound images previously analyzed;

    - at least one processor configured to:

        o detect and identify the presence of at least one landmark or the absence of landmarks in the current ultrasound image (13);
        o verify whether the identified landmarks in the current image are present in the landmark database (14);
        o if at least one of the identified landmarks in the current image are not present in the landmark database:

            ▪ triggering storage of the current ultrasound image in an image database;
            ▪ triggering storage in the landmark database of the at least one identified landmark which was not comprised in the landmarks database;
            ▪ verifying that all required landmarks have been stored in the landmark database and if at least one of the required landmarks is missing, triggering reception at least one additional current ultrasound image.

2. The device (1) according to claim **1,** wherein, for the detection and identification, the at least one processor is further configured to localize the landmarks in the current ultrasound image.

3. The device (1) according to either one of claims **1** or **2,** wherein the at least one processor is further configured to, before the detection of the landmarks, detect a region of interest (ROI) on the current ultrasound image comprising all the anatomical landmarks present in the current ultrasound image using a machine learning approach.

4. The device (1) according to claim **3,** wherein the at least one processor is further configured to localize the landmarks by using a machine learning approach receiving as input the region of interest of the current ultrasound image.

5. The device (1) according to claim **4,** wherein the at least one processor is further configured to compare an area of the region of interest to a predefined threshold and proceed to the localization of the landmarks in the region of interest only if the area of the region of interest exceeds the predefined threshold, otherwise triggering reception of at least one additional current ultrasound image.

6. The device (1) according to claim any one of claims **1** to **5,** wherein the at least one processor is further configured to select, for performing the detection and identification of the presence or the absence of landmarks in the current ultrasound image, a machine learning model previously trained on ultrasound images comprising at least part of the landmarks comprised in the current ultrasound image.

7. The device (1) according to any one of claims **2** to **6,** wherein the at least one processor is further configured to evaluate the quality of each localized landmark.

8. The device (1) according to claim **7,** wherein the at least one processor is further configured to trigger storage of the localized landmark in the landmarks database only if the localized landmark has at least a minimal predefined quality.

9. The device (1) according to any one of claims **1** to **8,** wherein, when all required landmarks have been acquired, the at least one processor is further configured to notify the user when all required landmarks have been acquired and stop triggering the reception of additional current ultrasound images.

10. The device (1) according to any one of claims **1** to **9,** wherein the detection and identification of the landmarks is performed by the at least one processor using a machine learning approach comprising at least one Convolutional Neural Network.

11. The device (1) according to any one of claims **1** to **10,** wherein the at least one processor is configured to iteratively perform the operations to do on a time sequence of ultrasound images.

12. The device (1) according to claim **11** wherein the at least one processor is configured to select a portion of the time sequence of ultrasound images comprising the highest number of localized landmarks having at least a minimal predefined quality.

13. A method for guiding a user in ultrasound assessment of an organ to perform a diagnostic or screening evaluation of said organ during a medical examination, said ultrasound assessment being based on ultrasound images, said method comprising:

    - receiving (11):

        o a current ultrasound image (21) resulting from at least a partial reflection of ultrasound by said organ;
        o at least two required landmarks used to perform diagnostic or screening evaluation of said organ;
        o a landmarks database, constructed from the ultrasound images previously analyzed;

    - detecting and identifying the presence of at least one landmark in the current ultrasound image;
    - verifying whether the identified landmarks in the current image are present in the landmark database;
    - if at least one of the identified landmarks in the current image are not present in the landmark database:

        ▪ triggering storage of the current ultrasound image in an image database;
        ▪ triggering storage in the landmark database of the at least one identified landmark which was not comprised in the landmarks database;
        ▪ verifying that all required landmarks have been stored in the landmark database and if at least one of the required landmarks is missing, triggering reception at least one additional current ultrasound image.

14. The method according to claim **13,** wherein, the detection and identification, further comprises the localization of the landmarks in the current ultrasound image.

15. A computer program comprising software code adapted to perform a method for guiding a user in ultrasound assessment of an organ according to any of claims **13** to 14 when said computer program is executed by a processor.

EP 4 144 301 A1

FIG. 1

**FIG. 2**

110 — Image

120 — Detect/identify presence or absence landmarks

130 — New landmarks identified?   N

140 — Trigger storage of the landmarks and of the image   Y

150 — All required landmarks identified?   N

160 — Trigger notification to the user   Y

**FIG. 3**

DL1

Im → Pre-T → Backbone CNN → FCNN →

View 1: Landmark 1
View 1: Landmark i
. . .

View 2: Landmark 1
View 2: Landmark j
. . .

View N: Landmark 1
View N: Landmark k
. . .

FIG. 4

FIG. 5

EP 4 144 301 A1

**FIG. 6**

DL2

Im → Pre-T → Backbone CNN → Neck CNN → Head CNN

$s_{11} * s_{12} * N_a * (N_c+6)$ propositions

$s_{21} * s_{22} * N_a * (N_c+6)$ propositions

→ Non-max suppression → Re-Scaling → Post-T → Landmarks identified and localized

(a)

(b)

**FIG. 7**

(a)

(b)

**FIG. 8**

(a)　　　　　　　　　　　　(b)

**FIG. 9**

(a)　　　　　　　　　　　　(b)

**FIG. 10**

**FIG. 11**

310 — Image

320 — Detect ROI comprising landmarks

330 — Is ROI acceptable? — N

Y

340 — Detect/identify and localize presence landmarks in ROI

350 — New landmarks identified? — N

Y

360 — Trigger storage of the landmarks and of the image

370 — All required landmarks identified? — N

Y

380 — Trigger notification to the user

**FIG. 12**

**FIG. 13**

410 — Image

420 — Determine view contribution

430 — Is there a dominant view? — N

Y

440 — Detect/identify/localize landmarks in ROI with a view's specific approach

450 — New landmarks identified? — N

Y

460 — Evaluate landmarks quality

470 — Is quality acceptable? — N

Y

480 — Trigger storage of the landmarks and of the image

**FIG. 14**

FIG. 15

**FIG. 16**

**FIG. 17**

**FIG. 18**

## EUROPEAN SEARCH REPORT

Application Number

EP 21 30 6211

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2014/219548 A1 (WELS MICHAEL [DE] ET AL) 7 August 2014 (2014-08-07) * paragraphs [0005], [0013], [0017], [0019], [0020] * ----- | 1-15 | INV. A61B8/08 A61B8/00 G06T7/00 |
| A | THOMAS KARAVIDES ET AL: "Database guided detection of anatomical landmark points in 3D images of the heart", BIOMEDICAL IMAGING: FROM NANO TO MACRO, 2010 IEEE INTERNATIONAL SYMPOSIUM ON, IEEE, PISCATAWAY, NJ, USA, 14 April 2010 (2010-04-14), pages 1089-1092, XP031693462, ISBN: 978-1-4244-4125-9 * abstract * ----- | 1-15 | |
| A | US 2020/234435 A1 (RAYNAUD CAROLINE DENISE FRANCOISE [FR] ET AL) 23 July 2020 (2020-07-23) * paragraphs [0044] - [0050] * ----- | 1-15 | |

TECHNICAL FIELDS SEARCHED (IPC)

A61B
G06T

The present search report has been drawn up for all claims

| Place of search | Date of completion of the search | Examiner |
|---|---|---|
| Munich | 15 February 2022 | Willig, Hendrik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

EPO FORM 1503 03.82 (P04C01)

33

## ANNEX TO THE EUROPEAN SEARCH REPORT
## ON EUROPEAN PATENT APPLICATION NO.

EP 21 30 6211

This annex lists the patent family members relating to the patent documents cited in the above-mentioned European search report.
The members are as contained in the European Patent Office EDP file on
The European Patent Office is in no way liable for these particulars which are merely given for the purpose of information.

15-02-2022

| Patent document cited in search report | | Publication date | Patent family member(s) | | Publication date |
|---|---|---|---|---|---|
| US 2014219548 | A1 | 07-08-2014 | CN | 103985147 A | 13-08-2014 |
| | | | US | 2014219548 A1 | 07-08-2014 |
| US 2020234435 | A1 | 23-07-2020 | CN | 110945560 A | 31-03-2020 |
| | | | EP | 3435324 A1 | 30-01-2019 |
| | | | EP | 3655917 A1 | 27-05-2020 |
| | | | JP | 6839328 B2 | 03-03-2021 |
| | | | JP | 2020527080 A | 03-09-2020 |
| | | | US | 2020234435 A1 | 23-07-2020 |
| | | | WO | 2019016064 A1 | 24-01-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 20305972 A